# EUROPEAN PATENT APPLICATION

(11) **EP 3 725 312 A1**
(43) Date of publication of application: **21.10.2020**
(21) Application number: 18886711.3
(22) Date of filing: 04.12.2018
(51) Int. Cl.: A61K 31/407, A61P 29/00

(54) **NEW CHEMICAL COMPOUND CONTAINING ACTIVE ENANTIOMER S-(-) KETOROLAC TROMETHAMINE FOR THE TREATMENT OF PAIN**

(30) Priority: 04.12.2017 MX 2017015623
(71) Applicant: Amezcua Amezcua, Federico, 28006 Madrid (ES); Amezcua Amezcua, Carlos, 28006 Madrid (ES)
(72) Inventor: GARCÍA ARMENTA, Patricia del Carmen, Zapopan Jalisco 45019 (MX)
(74) Representative: Gonzalez Palmero, Fé
(86) International application number: PCT/MX2018/000129
(87) International publication number: WO 2019/112413

(57) **Abstract**

The invention relates to a novel chemical compound characterized in that it contains the active enantiomer S-(-) ketorolac tromethamine combined with other pharmaceutically acceptable adjuvants or excipients, said compound being advantageous over other NSAIDs and, in particular, the known active principle ketorolac tromethamine, comprising a single isomer that is pure and has a better therapeutic index, retaining its therapeutic activity by improving its physicochemical properties, such as solubility or stability, which translates into a decrease in the dose administered, reducing direct gastrointestinal side effects and the time required for the development of the desired therapeutic action. The invention is suitable for use as a medication for the treatment of pain of moderate to high intensity and can be administered in any form in the dose range from 0.5 milligrams to 30 milligrams.

## Description

### FIELD OF THE INVENTION

The S-(-) ketorolac tromethamine is a novel chemical compound for the treatment of pain that is characterized as a non-steroidal anti-inflammatory (NSAID) drug from the family of the heterocyclic derivatives of acetic acid and used for its analgesic, antipyretic, and anti-inflammatory effect.

### BACKGROUND

There is no historical evidence of the use of the chemical compound S-(-) ketorolac tromethamine to treat pain. Nevertheless, we know that pain is the most common cause of medical consultations on a global level and has even increased in the past decade, being considered the fifth vital sign. The initiation of the treatment of pain depends on the intensity thereof, following the pain scale proposed by the WHO. On a global level, more than 30 million people take non-steroidal anti-inflammatory medication daily, 40% of whom are older than 60. Ketorolac, specifically, was introduced in the market more than a decade ago. Its use has increased rapidly, as have the associated gastrointestinal, renal, and hematological side effects.

The International Association for the study of Pain (IASP) defines it as "an unpleasant sensory and emotional experience associated with actual or potential tissue damage or described in terms of such damage." We can conclude then that pain is a complex response in the face of nociceptive stimuli that implies a negative affective factor, the suffering of which leads to a determined behavior.

Pain receptors are nerve endings that are regarded as specific by some investigators and nonspecific by others, there existing a third position that estimates that they are activated by low-intensity stimuli, meaning that higher-intensity pain can be transmitted by any receptor. The impulse is conducted by the dorsal root through sparsely myelinated A Delta fibers of small caliber (1 to 5 microns) and slow conduction (4 to 40 m/s) and through very thin unmyelinated C fibers (0.3 to 1.5 microns) with very slow conduction (0.4 to 2 m/s). They reach the posterior horn of the dorsal spine, to the *substantia gelatinosa* of Rolando in the form of layers. Some are collateral, forming the Lissauer tract that links the high medullary segments and underlying ones. Others cross toward the lateral region of the spine and ascend toward the thalamus through the direct spinothalamic and reticulospinal fascicles; the latter with connections to the brainstem. The direct spinothalamic tract projects over the ventroposterior nucleus of the thalamus (specific pain), while the reticular spinothalamic tract projects into the medial intralaminar nuclei (nonspecific pain). From there, the fibers travel toward the post-rolandic cortex (sensitive), but also to different nuclei, which explains the endocrine and vegetative (hypothalamus), emotive, and affective reactions (limbic system, reticular formation, lobe, frontal system) etc.

The pain receptors in the viscera appear to be different than in the periphery, reacting fundamentally to contraction and distension. The impulse travels through sympathetic nerves up to the lateral horn of the dorsal spine.

The pain message is subjected to peripheral, spinal, and supraspinal regulations. The prostaglandin, synthesized from the arachidonic acid derived from the phospholipids, appear to play an important role in the receptors. The synthesis is stimulated when there is tissue damage, and different enzymes intervene which can be interfered with by acetylsalicylic acid, indomethacin, phenylbutazone, corticoids, etc., with their subsequent analgesic effect.

At a medullar level, the myelinated Beta A thick fibers (6 to 7 microns), quick fibers (30 to 100 m/s) have a presynaptic inhibitory action that operates through axonal unions, while A delta and C fibers facilitate the passing of pain (gate control theory, Melzack and Wall, 1965).

A third control comes from the higher centers. Indeed, there is a descending path connected to the nucleus of the raphe magnus, periaqueductal grey matter, coeruleus and subcoeruleus nucleus, mediated by serotonin, and another from the cortex that accompanies the pyramidal tract, which intervene by moderating the pain impulse in the dorsal portion of the medulla.

The discovery of endogenous chemical substances which behave as neurotransmitters of pain has been significant. The "P substance", a polypeptide present in different areas of the central nervous system, among them the dorsal horn of the spinal cord, has been demonstrated to have a facilitating function (morphine blocks the pain by preventing its release). In the same manner, there are natural compounds having a chemical structure similar to that of morphine (morphinomimetical) called enkephalins and endorphins whose role, direct or indirectly, is to antagonize the transmission of pain. They have been related to opioid receptors (lipoproteins) present in the thalamus, periaqueductal grey matter, raphe nucleus, limbic system, locus coeruleus, medullary gelatinous substance, etc.

Pain is transmitted when the membrane of the neuron is depolarized. The action of analgesics is based in part on the capacity of these drugs to reduce the activation or sensitivity of the nociceptors by reducing the inflammation mediators present in the region (ex.: prostaglandins).

There has been an increase in the interest in the study of pain in recent years; the discovery of the descending inhibitory nervous pathways that originate in the brainstem and descend through the medulla, modulating the spinal nociceptive activity, represents one great advancement (Fields and Basbaum, 1978).

These descending pathways, like the spinal and supraspinal pathways, respond to opioids and other analgesics, as well as to physiological and experimental stimuli, including stress (Mayer and Liebeskind, 1974).

It has been speculated that the activation of this descending control system might be responsible for the analgesia induced by placebos and of the analgesic effects of acupuncture in some circumstances.

Even though the mechanisms and pathways of pain are better known today, it must be said that, besides the activation of the nociceptive pathways, individual perception of pain and the appreciation of its meaning are complex phenomena that involve psychological and emotional aspects (McGrath, 1990).

The perception of pain depends on complex interactions between the nociceptive impulses in the ascending pathways and the activation of descending inhibition systems. Knowing this gives us a basis for a more comprehensive and multimodal focus in the evaluation and treatment of the patients with pain and helps us confirm that clinical experiences that focus on a one-way approach are not the most appropriate.

Therefore, the individual management of pain must consider the state of the illness, concurrent medical conditions, characteristics of the pain, and psychological and cultural characteristics of the patient. A constant evaluation of the pain and the effectiveness of the treatment is also required.

As long as there is pain, the doctor must provide optimal alleviation, with routine evaluations and with one or more kinds of treatments.

The formulation of ketorolac in 10 mg tablets was approved in May 1997 in the US by the Food and Drug Administration (FDA). Ketorolac was approved by the FDA in 1999 in its parenteral presentation, and its ophthalmic presentation was approved in 2000.

Ketorolac belongs to the non-steroidal anti-inflammatory (NSAID) family, within the subclassification of arylpropionics. Other widely used NSAIDs belong to this group, such as naproxen, ibuprofen, and ketoprofen. Ketorolac shares the following structural characteristics with the drugs of its type: 1) Carboxyl acid group that is added to the site of action, 2) phenyl group, and 3) flat structure formed by a nitrogen that provides stability in the union with cyclooxygenase (COX), the enzyme complex over which it acts, like the rest of the NSAIDs. The cyclooxygenase is an enzyme that has two different isoforms (COX-1 and COX-2), and it is responsible for synthesizing prostaglandins (PG) from arachidonic acid. The tissues in which each isoform is expressed are different: COX-1 is a constitutive part of the organism - among which the parietal, cardiac, renal and epithelial cells stand out - and has the purpose of synthesizing PG protectors of gastric mucosa, of electrolytic homeostasis in cases of hypovolemia, PGs involved in the vasodilation and those which facilitate platelet aggregation. On the other hand, COX-2, besides being constitutive in lesser amounts, is also induced by inflammatory processes, since in addition to the synthesis of the prostaglandins, it is a catalyst of the production of prostacyclin and thromboxane responsible for the vasoconstriction/ vasodilation regulation, fibrinolysis, sensitization of peripheral nociceptive receptors, and other events that occur during inflammation. Since it is not selective for some of the isoforms, Ketorolac inhibits the formation of pro-inflammatory PGs and PGs in peripheral nociceptors (anti-inflammation and analgesia: desired pharmacological effects) 7, 9, 10 such as the production of "protective" PGs from COX-1. This phenomenon explains many of the adverse side effects associated with the administration of ketorolac.

The pharmacokinetics of a medication are determinative for the guidelines in the recommendations for its proper use, with parameters such as absorption, maximum concentration, metabolism, and excretion being considered for this purpose. In the case of ketorolac, the onset of the analgesic effects is observed 30 minutes after its administration, in any presentation, reaching a maximum level around the first and second hour with intravenous and intramuscular use and 3 hours after oral administration, whether in the form of tablets or capsules. When combined with food with abundant fat, oral absorption may be delayed, which manifests as inadequate control of pain, so it is recommended that patients avoid those kinds of food at the time of the oral administration of that medication. As regards its distribution, ketorolac bonds with plasmatic proteins 99% of the time; this bond is weak, however, so the concomitant use of another NSAID can have the effect of displacing ketorolac, increasing the plasmatic concentration of the free drug and enhancing the adverse gastrointestinal effects. Once the ketorolac has reached the bloodstream, the drug is metabolized in the liver, resulting in inactive metabolites, mainly by conjugation (21%). In patients with liver disease, the half-life of the drug increases, so the dose must be adjusted. Ketorolac is eliminated renally and is excreted as the unaltered drug (60.2%), as a conjugated metabolite (21.9%), or as a hydroxylated metabolite (11.5%). The fact that the drug is excreted without any change in most cases, and given the possible side effects at a renal level means that the dose should be modified in patients with mild renal insufficiency and older people, and it is also contraindicated for those suffering from moderate or severe renal failure, with a clearance of less than 30 mL/min.

At the clinical level, there are many non-steroidal anti-inflammatory analgesic drugs (NSAIDs) that are employed therapeutically to alleviate pain. Many of these NSAIDs are administered in the form of their racemic mixtures, in which only one of the components of the racemic mixture is the active component or the one generating the desired pharmacological effect, while the other enantiomer does not produce the desired effects and may even be responsible for side effects.

Ketorolac tromethamine is an analgesic that acts through the inhibition of cyclooxygenase enzymes, preventing the synthesis of the prostaglandins.

The novel chemical compound described in the present invention, S-(-)-ketorolac tromethamine, is a non-steroidal anti-inflammatory (NSAID) from the family of the heterocyclic derivatives of acetic acid and is used as an analgesic, antipyretic, and anti-inflammatory drug. The enantiomer S (-) is the most active; it has been determined that this enantiomer of the racemic mixture is the one that basically has the analgesic effect, which is almost double that of the racemic form and approximately 60 times more potent, thereby enabling the dose to be reduced by up to 50% and thus reducing the risk of severe side effects that are produced due to the chronic consumption of the medications based on the current ketorolac racemic salts.

### OBJECT OF THE INVENTION

The novel chemical compound with the name S-(-) ketorolac tromethamine, constituted by the enantiomer S(-), allows for a reduction in the dose, improves the pharmacological potency, and additionally improves the therapeutic index without sacrificing the efficacy of the drug when compared with the chronic consumption of medications based on the current ketorolac tromethamine racemic salt.

### BRIEF DESCRIPTION OF THE FIGURES

FIGURE 1. Antinociceptive efficacy in DRC (oral route)
FIGURE 2. Pharmacological potency of ASA, morphine, rac-ketorolac, and s-ketorolac
FIGURE 3. Maximum effect (Emax) on CT of maximum doses (oral route)
FIGURE 4. Global effect of maximum dose (for 4 hours)
FIGURE 5. Fixed effect 4 hours after administrating maximum dose
FIGURE 6. Effective dose 50 in rats, oral route
FIGURE 7. Lethal dose in 50% of the rat population, oral route
FIGURE 8. Therapeutic index in rats, oral route

### DETAILED DESCRIPTION OF THE INVENTION

The S-(-)-ketorolac tromethamine is a novel chemical compound that is characterized as a non-steroidal anti-inflammatory (NSAID) from the family of the heterocyclic derivatives and is used as an analgesic, antipyretic, and anti-inflammatory drug. Unlike ketorolac tromethamine, this chemical compound is constituted by the enantiomer S(-), which is the most active, 60 times more active than the R (+) form. The consequence of this is that, unlike ketorolac tromethamine, the dose administered can be reduced, thus decreasing the direct gastrointestinal side effects and the time required for the development of the desired therapeutic effect.

The S-enantiomers of the derivatives 2-arylpropionic combined with tromethamine salt share all of the benefits of the non-selective COX inhibitors, with easy absorption and fewer side effects than its main compounds, and turn out to be an excellent option for the treatment of moderate to severe pain.

The chemical structure of the previously known ketorolac racemic salt is as follows:

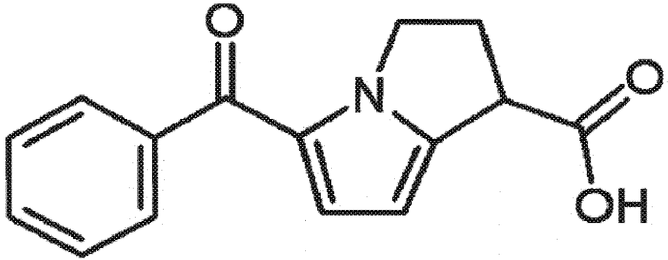

Meanwhile, the chemical structure of the novel chemical compound S-(-)-ketorolac tromethamine that is described in the present invention is as follows:

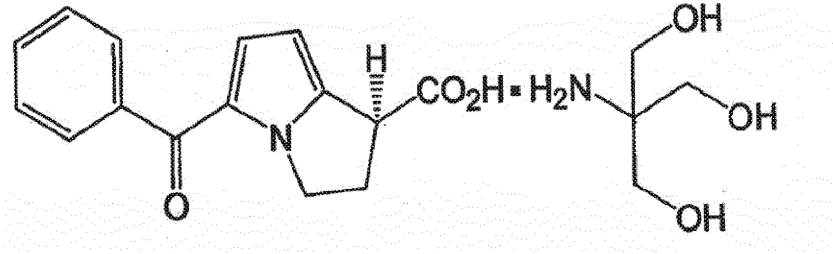

And its nomenclature is:
- 2-amino-2-hydroximethyl-1,3 propanediol (1:1) S-(-)-5-benzoyl-2,3-dihydro-1H-pyrrolizine-1-carboxylate;
- S-(-)-benzoyl-2,3-dihydro-1H-pyrrolizine-1-carboxylic acid salt with 2-amino-2-hydroximethyl-1,3-propanediol (1:1).

The results of the preclinical trial titled "Pharmacological comparison of the antinociceptive effects of S-(-)-ketorolac with a prototypical analgesic of the opioid group and with a prototypical analgesic of the group of the NSAIDs," conducted by Dr. Francisco Javier Lopez Muñoz and dated September 20, 2017, showed that the analgesics included in this trial had adequate antinociceptive effects in experimental conditions of gouty arthritis-type pain in lab animals: male Wistar rats weighing between 180 and 200 grams. The prototypical analgesic of the opioid group that was compared with S-(-)-ketorolac tromethamine was morphine, and the prototypical analgesic of the group of the NSAIDs was acetylsalicylic acid. In order to determine and compare the antinociceptive effects of these analgesics the preclinical experimental model of PIFIR "pain-induced functional impairment model in the rat" was used. Based on this, it was demonstrated that: (i) with regard to the pharmacological efficacy of the S-(-)-ketorolac tromethamine, it exhibited similar efficacy to that exhibited by rac-ketorolac and to the prototypical opioid analgesic, morphine, but S-(-)-ketorolac tromethamine exhibited much more efficacy than that exhibited by the prototypical NSAID agents such as acetylsalicylic acid, as is demonstrated in the following table:

| ANALGESIC | DOSE (mg/kg. oral route) | MAXIMUM EFFICACY (ABC: X ± E.E.) | RELATIVE EFFICACY |
|---|---|---|---|
| rac-ketorolac | 10.0 | 324.6 ± 7.8 | 1.0 |
| | 3.2 | 310.5 ± 13.5 | 1.0 |
| Morphine | 177.8 | 310.8 ± 8.9 | 1.0 |
| Acetylsalicylic Acid | 562.3 | 226.6 ± 11.7 | 0.70 |

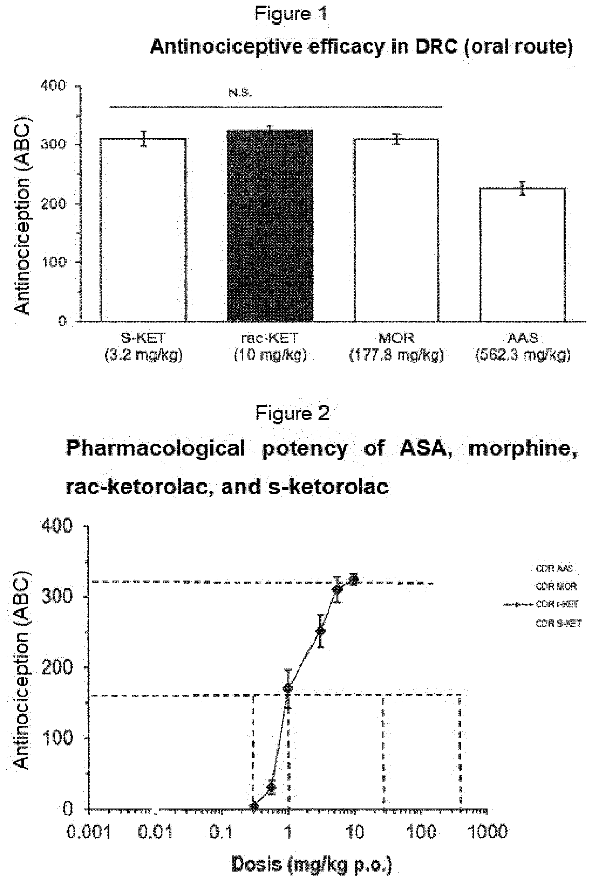

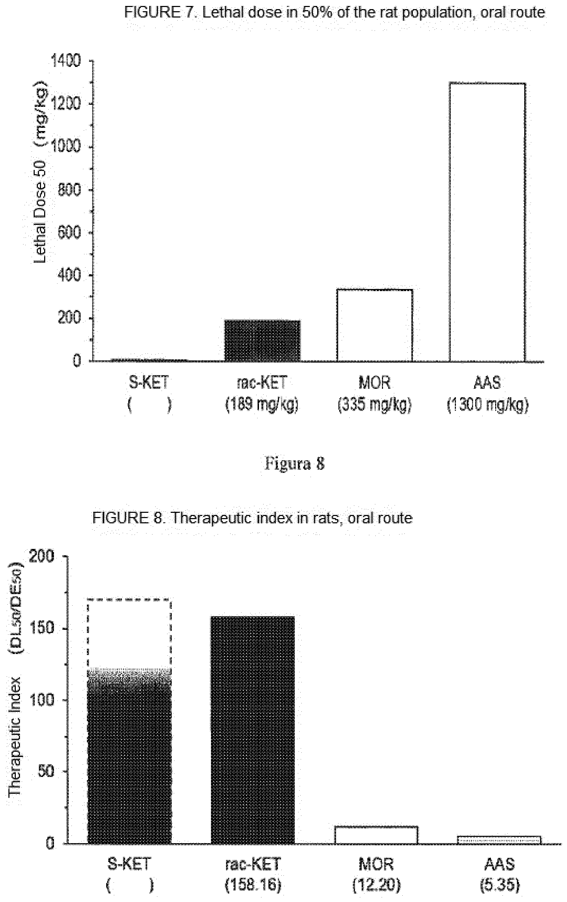

Also, see graphic in Fig. 1/8.- The comparison of the antinociceptive efficacy of the 4 analgesics determined in the CDR, and the dose which generates them can be seen. (N.S.= No significant difference and numeric analysis); (ii) with regard to the pharmacological potency, S-(-)-ketorolac tromethamine turned out to be more potent than morphine, the prototypical opioid analgesic, and the NSAID acetylsalicylic acid, when comparing doses needed to generate 50% of the most effective effect generated in experimental conditions. Therefore, S-(-)-ketorolac tromethamine was 3.3 times more potent than rac-ketorolac, 82 times more potent than morphine, and 1037 times more potent than acetylsalicylic acid, as is shown in the following table:

| ANALGESIC | ED₆₀ (mg/kg, oral route) (with regard to Emax: 324 au) | RELATIVE POTENCY (relative to S-ketorolac) |
|---|---|---|
| | 0.36 ± 1.0 | --- |
| rac-Ketorolac | 1.19 ± 1.2 | -3.3 |
| Morphine | 29.35 ± 1.1 | -81.5 |
| Acetylsalicylic Acid | 373.3 ± 1.7 | -1,036.9 |

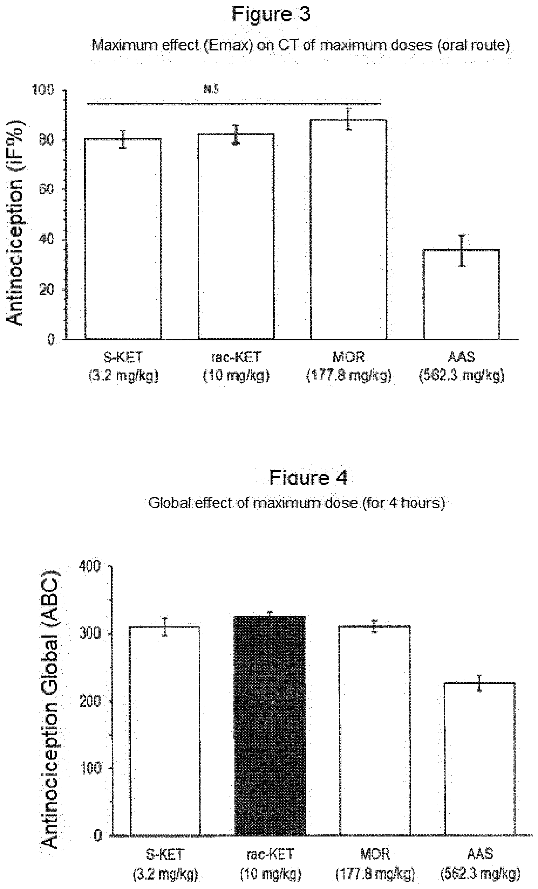

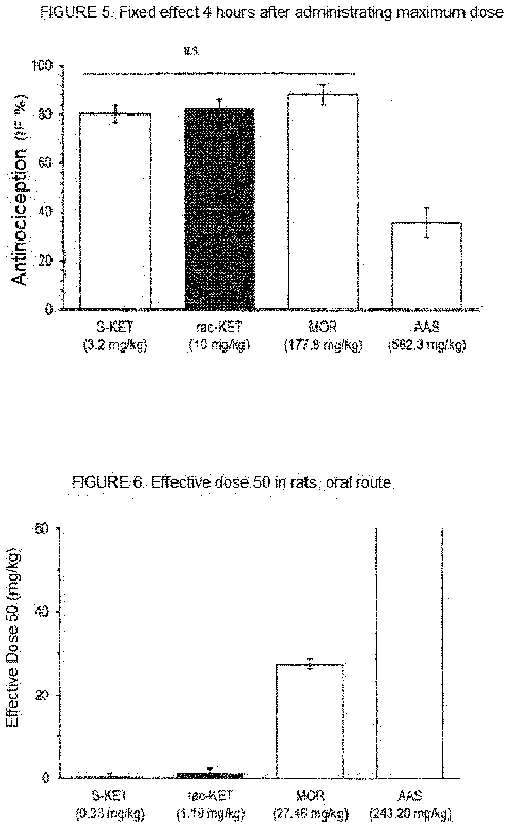

Also, see graphic in Fig. 2/8.- Shown is the calculation of the ED₅₀ and a comparison of the antinociceptive potency of the 4 analgesics; (iii) with regard to latency in reaching the Emax in the temporal curve, S-(-)-ketorolac tromethamine exhibited a latency similar to that exhibited by rac-ketorolac, but less than that exhibited by morphine; that is, it reaches its Emax faster than morphine administered orally. However, the acetylsalicylic acid exhibited the least latency in reaching its Emax, even though that Emax was smaller than those exhibited by the other analgesics. See graphic in Fig. 3/8.- This shows the maximum effects exhibited by the highest doses that it is possible to administer of each one of the analgesics by oral route; (iv) With regard to the Emax reached in the temporal curve, S-(-)-ketorolac tromethamine exhibited an Emax similar to that exhibited by rac-ketorolac and morphine, but this Emax was higher than that exhibited by acetylsalicylic acid. See graphic in Fig. 4/8.- Shown are the global antinociceptive effects achieved by the analgesics upon administration of larger doses by oral route; (v) with regard to a fixed effect 4 hours after administration, the effect produced by S-(-)-ketorolac tromethamine was the same as that exhibited for rac-ketorolac and by morphine but higher than that exhibited by acetylsalicylic acid. See graphic in Fig. 5/8.- This shows the antinociceptive effects exhibited after 4 hours of the administration of the analgesics in the higher doses through oral route; (vi) with regard to the effective dose 50, this turned out to be less than the rest of the active substances being studied. See graphic in Fig. 6/8.- Shown are the effective doses 50 (ED₅₀) that are particular to each analgesic (taking into account its own efficacy) to produce 50% of its own maximum effect obtained in the PIFIR experimental model in rats by oral route; (vii) With regard to lethal dose 50, S-(-)-ketorolac tromethamine exhibited a LD 50 smaller than the acetylsalicylic acid, but larger than the one reported for rac-ketorolac and morphine, as can be seen in the graphic of Fig. 7/8, which shows a lethal dose 50 (LD₅₀) of each analgesic to produce death in 50% of the rat population upon administration of every one of the analgesics; (viii) due to the small magnitude of the ED 50 and the large magnitude of the LD 50 of S-(-)-ketorolac tromethamine, a very high and favorable therapeutic index was found with regard to the therapeutic index of rac-ketorolac, of morphine, the opioid prototype, and of the acetylsalicylic acid, the NSAID prototype, as is described in the graphic of Fig. 8/8, which shows the therapeutic index of each analgesic administered by oral route in rats.

As a consequence of the above, it was concluded in that preclinical trial that very adequate characteristics for efficacy, potency, Emax, Emax latency, fixed effects, and toxic effects were established with the therapeutic index for S-(-)-ketorolac tromethamine, the active enantiomer of ketorolac, compared to the racemic mixture of ketorolac with morphine, the opioid prototype, and acetylsalicylic acid, the NSAID prototype.

The following conclusions were drawn from the results of the cited preclinical trial: The chemical compound S-(-)-ketorolac tromethamine turned out to be an analgesic with very adequate characteristics for efficacy, potency, Cmax, fixed and toxicity effects with the therapeutic index for S-(-)-ketorolac, the active enantiomer of ketorolac, when compared to the racemic mixture of ketorolac tromethamine with morphine, the opioid prototype, and acetylsalicylic acid, the NSAID prototype.

Now, if we compare the novel chemical compound described in the present invention with ketorolac tromethamine, it can be said that the latter is a potent analgesic but that its most frequent side effects are directly related to the pharmacological actions for this type of drugs. In particular, they produce effects over the gastrointestinal tract and on the renal and hematological function and inhibit platelet aggregation and can incite the formation of gastric ulcers.

Scientific evidence shows that the risk of developing severe complications involving peptic ulcers (in particular, high digestive hemorrhaging) is considerably higher with the use of ketorolac tromethamine than with other non-steroidal anti-inflammatory drugs, and the increase in the risk can be particularly substantial when it is used outside the conditions of use that are currently authorized. The chemical compound ketorolac tromethamine is a racemic mixture of the enantiomers (-) S and (+) R, the first of which is the one that possesses analgesic activity.

The intention behind the strategy of reevaluating the racemic drug and segregating the beneficial part of a non-steroidal anti-inflammatory analgesic such as S-(-)-ketorolac was to develop a novel chemical compound with a single isomer that is pure and has a better therapeutic index than the mixture formulated as a racemate of ketorolac tromethamine.

This novel chemical compound that is protected in the present invention has several substantial advantages over its predecessor, ketorolac tromethamine, since it retains its therapeutic activity while improving its physicochemical properties, such as solubility or stability, which translates into better solubility and less time required to obtain a therapeutic response.

This novel chemical compound comprises the active enantiomer S-(-) ketorolac tromethamine in combination with pharmaceutically acceptable excipients or adjuvants to be prescribed or administered in any pharmaceutical form to the patient as a medication for the treatment of pain of moderate to high intensity in the dose range from 0.5 milligrams to 30 milligrams.

## Claims

1. A novel chemical compound, **characterized in that** it contains an active enantiomer, S-(-) ketorolac tromethamine, which is **characterized by** the following structure and nomenclature: 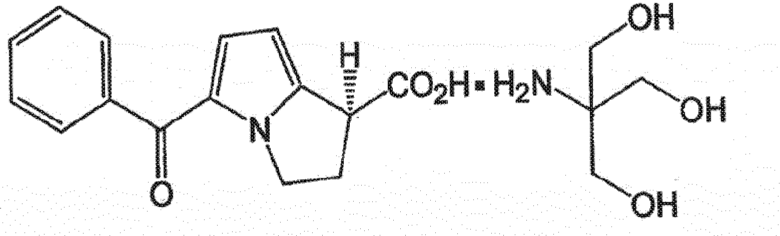
• 2-amino-2-hydroximethyl-1,3 propanediol (1:1) S-(-)-5benzoyl-2,3-dihydro-1H-pyrrolizine-1-carboxylate;
• S-(-)-benzoyl-2,3-dihydro-1H-pyrrolizine-1-carboxylic acid salt with 2-amino-2-hydroximethyl-1,3-propanediol (1:1).

2. The novel chemical compound as set forth in claim 1, **characterized in that** it comprises the active enantiomer S-(-) ketorolac tromethamine in combination with other pharmaceutically acceptable excipients or adjuvants.

3. The novel chemical compound as set forth in claim 1, **characterized in that** it comprises a single isomer that is pure and has a better therapeutic index than its predecessor, the active substance having the name ketorolac tromethamine.

4. The novel chemical compound as set forth in claim 1, **characterized in that** it retains its therapeutic activity improving its physicochemical properties, such as solubility or stability, which results in fewer direct gastrointestinal side effects and less time required for the development of the desired therapeutic action, advantages that are significant when compared with other NSAIDs and in particular with its predecessor, the known active substance ketorolac tromethamine.

5. The novel chemical compound as set forth in claim 1, **characterized in that** it is used as a medication for the treatment of moderate to severe pain.

6. The novel chemical compound as set forth in claim 5, **characterized in that** it can be administered in any form.

7. The novel chemical compound as set forth in claim 5, **characterized in that** it can be administered in the dose range that can vary from 0.5 milligrams to 30 milligrams.
